Europäisches Patentamt

European Patent Office

Office européen des brevets

(11) Numéro de publication: **0 171 331**
A1

## (12) DEMANDE DE BREVET EUROPEEN

(21) Numéro de dépôt: **85401560.9**

(22) Date de dépôt: **31.07.85**

(51) Int. Cl.⁴: **A 61 K 31/49,** A 61 K 47/00, A 61 K 9/08

(30) Priorité: **03.08.84 FR 8412307**

(43) Date de publication de la demande: **12.02.86 Bulletin 86/7**

(84) Etats contractants désignés: **BE CH DE GB IT LI LU NL SE**

(71) Demandeur: **LABORATOIRE VAILLANT DEFRESNE, 65-77 rue Falguière, F-75739 Paris Cedex 15 (FR)**

(72) Inventeur: **Versini, François, 51, rue de la Glacière, F-75013 Paris (FR)**

(74) Mandataire: **Orès, Bernard et al, Cabinet ORES 6, avenue de Messine, F-75008 Paris (FR)**

(54) **Nouvelle composition liquide à base de formiate de quinine.**

(57) La présente invention est relative à une nouvelle composition à base de formiate de quinine.

Cette composition se caractérise en ce qu'elle contient par rapport à la quinine présente entre 30 et 100% d'un acide-alcool.

Application: antipaludéen.

La présente invention est relative à de nouvelles compositions à base de formiate de quinine. La quinine, principal alcaloïde extrait des écorces de quinquina, est un antipaludique majeur surtout dans les formes malignes du paludisme, car la rapidité de son action la fait employer, de préférence aux antipaludéens de synthèse, contre les formes rares et les accès pernicieux. La quinine forme de nombreux sels qui peuvent être classés en sels dits "neutres", solubles dans l'eau, mais acides et irritants pour les muqueuses, et très douloureux à l'injection, et en sels dits "basiques" qui sont peu à très peu solubles dans l'eau, mais mieux tolérés par l'organisme.

A la première catégorie appartient par exemple le chlorhydrate, le bromhydrate ou le sulfate de quinine, tandis que le formiate de quinine appartient à la seconde catégorie. Sa solubilité est d'environ 5 % à 15°C, donc trop faible pour envisager l'administration par injections (intraveineuses ou intramusculaires), lesquelles injections sont souvent indispensables lors de crises graves.

Les recherches en vue d'une meilleure solubilisation ont abouti à l'adjonction de l'uréthane, produit parfaitement solubilisant permettant l'obtention de solutés injectables à haute teneur en quinine.

Toutefois des expériences récentes ont montré de très graves risques liés à l'utilisation de l'uréthane : le produit s'est avéré néphrotoxique, hépatoxique et aurait en plus une action mutagène et cancérigène. Ces graves risques de santé ont été décrits par de nombreux auteurs et notamment par :

- BATEMAN A.J. (The mutagenic action of urethane, Mut. Res, 1976, 39, 75-96)
- KLATSKIN G. (Toxic and drug - induced hepatitis, "Diseases of the liver", p. 604-710, 1975, Lippincott)
- BRODSKY I. et coll. (Amer. J. Med., 1961, 30, 976-980) et beaucoup d'autres.

2

La présente invention s'est par conséquent donné pour but de pourvoir à une forme soluble de quinine d'une inocuité totale et ne présentant pas les risques et les graves inconvénients de l'uréthane.

La présente invention a pour objet une nouvelle composition à base de formiate de quinine, caractérisée en ce qu'elle contient par rapport à la quinine présente entre 30 et 100 % (poids sur poids) d'un acide-alcool.

Selon un mode de réalisation avantageux de l'objet de la présente invention, l'acide-alcool est constitué par l'acide lactique et/ou par l'acide tartrique. Ces deux acides, physiologiquement parfaitement compatibles et d'une tolérance depuis longtemps éprouvée, solubilisent de manière surprenante le formiate de quinine et rendent possible l'utilisation de ce sel sans danger quelque soit le mode d'administration choisi.

Selon un autre mode de réalisation de l'objet de l'invention la solution de formiate de quinine contient en outre un antioxydant et de l'acide formique entre 0,2 à 3 % (poids/poids) par rapport au formiate de quinine. La présence de ce dernier assure une meilleure stabilité de la solution en fonction du temps.

Outre les dispositions qui précèdent, l'invention comprend encore d'autres dispositions qui ressortiront de la description qui va suivre, qui se réfère à des exemples de formulation des compositions conforme à la présente invention.

Il doit être bien entendu, toutefois, que ces exemples sont donnés uniquement à titre d'illustation de l'objet de l'invention mais n'en constituent en aucune manière une limitation.

3

Exemples de formulation

Exemple 1

| | |
|---|---|
| Formiate de quinine | 84 kg |
| Acide lactique | 33,6 kg |
| Sulfite de Na | 0,202 kg |
| Eau pour préparation injectable   q.s.p. | 336 l |

Exemple 2

| | |
|---|---|
| Formiate de quinine | 84 kg |
| Acide lactique | 33,6 kg |
| Acide formique | 1,74  kg |
| Sulfite de Na | 0,202 kg |
| Eau pour préparation injectable    q.s.p. | 336 l |

Exemple 3

| | |
|---|---|
| Formiate de quinine | 84 kg |
| Acide tartrique | 84 kg |
| Sulfite de Na | 0,202 kg |
| Eau pour préparation injectable    q.s.p. | 336 l |

Les solutions stériles conforme à la présente invention sont réparties en ampoules de 1 ml, 2 ml et 4 ml.

Ainsi que cela ressort de ce qui précède, l'invention ne se limite nullement à ceux de ses modes de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

# REVENDICATIONS

1. Nouvelle composition liquide, antipaludique, à base de formiate de quinine, caractérisée en ce qu'elle contient par rapport à la quinine présente entre 30 et 100 % (poids sur poids) d'un acide-alcool, constitué par l'acide lactique et/ou par l'acide tartrique.

2. Composition selon la revendication 1, caractérisée en ce que la solution de formiate de quinine contient en outre un antioxydant et de l'acide formique entre 0,2 à 3 % (poids/poids) par rapport au formiate de quinine.

0171331

**RAPPORT DE RECHERCHE EUROPEENNE**

Numero de la demande

EP 85 40 1560

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| Y | CHEMICAL ABSTRACTS, vol. 76, no. 7, 14 février 1972, page 9, no. 30537s, Columbus, Ohio, US; N. YAMANAKA et al.: "Effect of quinoform on the regulation of mitochondrial respiration" & IGAKU NO AYUMI 1971, 76(13), 919-20 * Résumé * | 1-2 | A 61 K 31/49<br>A 61 K 47/00<br>A 61 K 9/08 |
| Y | US-A-4 287 214 (E.J. VAN SCOTT) * Colonne 12, lignes 56-63; revendication * | 1-2 | |
| Y | EP-A-0 015 721 (E.S. FOX) * Revendications 1,2,6 * | 1-2 | |

----

| | | | DOMAINES TECHNIQUES RECHERCHES (Int. Cl.4) |
|---|---|---|---|
| | | | A 61 K |

Le présent rapport de recherche a été établi pour toutes les revendications

| Lieu de la recherche | Date d'achèvement de la recherche | Examinateur |
|---|---|---|
| LA HAYE | 11-10-1985 | BERTE M.J. |